# EUROPEAN PATENT SPECIFICATION

(11) **EP 3 482 778 B1**
(45) Date of publication and mention of the grant of the patent: **16.09.2020**
(21) Application number: 18187284.7
(22) Date of filing: 03.08.2018
(51) Int. Cl.: A61L 2/26, A61L 2/07, B08B 3/04, B65G 1/10

(54) **MACHINE FOR TREATING OBJECTS, IN PARTICULAR WASHING, THERMAL DISINFECTION AND/OR STERILIZATION OF OBJECTS**
MASCHINE ZUR BEHANDLUNG VON OBJEKTEN, INSBESONDERE ZUM WASCHEN, ZUR THERMISCHEN DESINFEKTION UND/ODER ZUM STERILISIEREN VON OBJEKTEN
MACHINE DE TRAITEMENT D'OBJETS, EN PARTICULIER LE LAVAGE, LA DÉSINFECTION THERMIQUE ET/OU LA STÉRILISATION D'OBJETS

(30) Priority: 04.08.2017 IT 201700090475
(43) Date of publication of application: 15.05.2019
(73) Proprietor: Steelco S.p.A., 31039 Riese Pio X (IT)
(72) Inventor: Capovilla, Ivone, 31037 Loria (TV) (IT)
(74) Representative: Petraz, Gilberto Luigi

(56) References cited:
- WO-A1-2014/116947
- WO-A1-2015/145383
- WO-A2-2017/203036

## Description

### FIELD OF THE INVENTION

The present invention concerns a machine for treating objects, in particular a so-called "tunnel" treatment machine, in which successive steps of the treatment of objects can be carried out in one or more treatment chambers in which relative treatment zones are disposed one after the other.

By the term treatment it is possible to understand "in their entirety", both pretreatment operations of the objects, such as pre-washing, with hot or cold water and/or with detergents or other chemical products, and also washing operations proper, and also drying operations. Also included in the same term "treatment" are thermal disinfection, sterilization, generally in an autoclave, and decontamination by means of particularly aggressive and dangerous decontaminating substances, detergents and/or chemical agents, such as for example peracetic acid.

By way of example, the objects that can be treated in the treatment machine in question can be instruments used in the health sector, in the laboratory, for analysis or for research, instruments used in the pharmaceutical sector, or instruments in the medical sector, surgical instruments or comparable or similar instruments, without excluding, however, the application of the present invention to the treatment of objects in general.

### BACKGROUND OF THE INVENTION

It is known to make machines for treating objects, generally contained in one or more object-holder containers, such as racks or suchlike, which are fed inside at least one suitable treatment chamber, by feed means, for example a belt, where the desired treatment cycle is performed.

Normally, at least one door is provided, associated with an entrance aperture, which is mobile and driven by an opening and closing mechanism to assume a high or open position in which it allows the entrance of the objects to be treated in the treatment chamber, and a low or closed position in which, in cooperation with fluidic sealing means, it seals the washing chamber from the outside.

In single-door treatment machines, the entrance and exit of the containers and of the treated objects contained therein take place through the same door. In pass-through treatment machines, two doors are provided, that is, one door associated with an entrance aperture and one door associated with an exit aperture, normally in a position opposite the entrance aperture, respectively for the entrance and exit of the containers and of the treated items contained therein.

Inside the machine's treatment chamber, several object-holder containers can also be inserted, located in sequence one after the other in the chamber.

In the case of a single-door treatment machine, a rollerway is provided on which the object-holder containers are rested and situated in correspondence with the door of the machine. From the door, therefore, the containers enter with the objects to be treated and the containers with the treated objects come out.

In the case of a pass-through treatment machine, generally two rollerways are provided, positioned on diametrically opposite sides of the machine, of which one side has an entrance door for the containers with objects to be treated and, the opposite side, has an exit door of the containers with the treated objects.

In particular, the treatment machines configured for sterilization are generally machines with a deep treatment chamber, for example even a few meters long, therefore, in such machines, multiple object-holder containers disposed in sequence are loaded. In the sterilization treatment relatively high temperatures are reached, for example up to about 140°C, therefore, it is good practice if the sterilization machine does not have, inside the treatment chamber, automatic movement means for the object-holder containers and/or sensors to detect their position.

To load the treatment chamber and start the intended treatment cycle, the object-holder containers are thrust and translated one after the other through the loading door of the treatment chamber. This can happen both for single-door machines and for pass-through machines.

In the case, for example, of two containers located in sequence, a first container will be inserted into the chamber, then, once the insertion is completed, a second container will be positioned after the first container, which, following the thrust of the second container, will occupy a bottom zone of the chamber.

Once the containers have been inserted in sequence, the chamber is closed and the treatment cycle begins.

In the event that, for any reason whatsoever, for example a fault, an alarm or other, the intended cycle is not completed and it becomes necessary to remove the two containers located in the chamber, the loading door is opened so that they can be extracted.

It should be noted that in the case of premature interruption of a treatment cycle, for example due to a fault or alarm, even in a pass-through machine it is necessary to unload the containers from the loading door, as the unloading door automatically blocks. This blocking of the unloading door is mainly due to the fact that the zone of the treated objects, located downstream of the machine, must not in any way enter into communication with the zone of the objects still to be treated.

If, on the contrary, the cycle proceeds normally, the containers located in sequence are then unloaded, in the pass-through machine, from the exit door which is situated on an opposite side of the machine with respect to the entrance door. Therefore, the normal loading and unloading of the containers into/from pass-through machines is done in a same direction and in a same direction of translation.

In all tunnel machines, both single-door and pass-through, the operations to extract the object-holder containers can be very difficult, especially for object-holder containers located farther down the chamber with respect to the side from which they will be unloaded.

Normally, in order to extract a container that is in proximity to the bottom of a treatment chamber, an operator has to enter into the chamber until he can grasp the object-holder container and pull it toward him in order to remove it. This operation, as can be imagined, proves rather difficult, since the environment of the treatment chamber, as we said above, can be extremely hot and furthermore the container can have a certain weight, since it is loaded with the objects to be treated.

In a treatment machine provided with a treatment chamber in which several object-holder containers disposed in sequence are positioned, therefore, it can be extremely difficult and not very safe for operators to remove the object-holder containers.

This problem can occur as mentioned both in the case of a single-door machine and a pass-through machine, in which the cycle is prematurely interrupted, therefore essentially when the containers are unloaded from the same side as they are loaded.

For a pass-through machine, the container inserted first in the chamber will be the one located closest to the exit door, while the container inserted last, at the end of a normal treatment cycle, will be at the bottom of the chamber, so it will present greater difficulties in extraction.

Substantially, therefore, both for pass-through and single-door type machines, in the case of at least two containers located in sequence, the container which is farther to the bottom of the treatment chamber has obvious difficulties in extraction, whether this extraction is carried out on the loading side, or on the unloading side.

A known device to introduce and extract containers with respect to a sterilization machine and sterilization machine comprising said device are described for example in the document WO-A-2015/145383. Document WO-A-2017/203036 describes another known machine for treating, in particular sterilizing, objects. The document WO-A-2014/116947 describes a known inventory system with connectable inventory holders.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain a machine for treating objects, in particular for washing, thermal disinfection and/or sterilization of objects, which can overcome at least one of the disadvantages of the prior art.

In particular, one purpose of the present invention is therefore to provide a machine for treating objects which allows the automatic attachment of several object-holder containers disposed in sequence upon their insertion into the treatment chamber, so that, acting on the slider closest to the loading door, or unloading door, in the direction opposite or concordant to the direction of insertion, all the object-holder containers present in the chamber can be extracted, and therefore also the object-holder container located at the bottom of the treatment chamber and in the farthest position from the loading door or the unloading door, thanks to the fact that it is attached to the object-holder container closest to the loading or unloading door.

Another purpose of the present invention is to provide a machine for treating objects which has a device of a mainly mechanical type to automatically attach the sliders upon their insertion.

Another purpose of the present invention is to provide an effective and automatic reciprocal attachment apparatus for several object-holder containers, which allows to translate the object-holder containers simultaneously at least in the step where they are extracted from the treatment chamber and at least as far as the loading door of any machine for treating objects.

Another purpose of the present invention is to perfect a quick, automatic and safe method for the reciprocal attachment of several object-holder containers.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a machine for treating objects, in particular washing, thermal disinfection and/or sterilization of objects according to the invention comprises at least a treatment chamber configured to house a plurality of object-holder containers located in sequence. The treatment chamber comprises an entrance aperture and lateral walls.

The machine comprises a first object-holder container comprising opposite sides and at least a releasable connection device comprising: at least one rotatable arm provided on at least one of said opposite sides of said first object-holder container and configured to pass, by means of activation means positioned in the treatment chamber, from a first inactive release position to a second active attachment position; and wheel or other rolling element.

The machine further comprises a second object-older container comprising opposite sides and at least one pin protruding from one side of said second object-holder container and suitable to be engaged by said rotatable arm in the second active attachment position.

The activation means positioned in the treatment chamber comprises at least a cam disposed on the internal surface of the lateral walls of the treatment chamber.

The releasable connection device, the pin and the activation means are configured to cooperate, so that, inserting the second object-holder container in the treatment chamber after at least a first object-holder container already inserted in said chamber and moving said second object-holder containers toward the inside of the chamber, said releasable connection device is automatically activated by said activation means, by thrusting said second object-holder container against said first object-holder container, causing the rotation of said rotatable arm from said first inactive release position to said second active attachment position thanks to the sliding of said wheel or other rolling element along said cam, in order to reciprocally connect said object-holder containers, so as to allow a simultaneous extraction thereof, acting on the first or last object-holder container inserted.

According to another aspect of the invention, the machine comprises means to de-activate the releasable connection device, so as to automatically separate the object-holder containers in a step of their extraction from the treatment chamber.

The de-activation means could be positioned in proximity to an entrance and/or exit aperture of the treatment chamber.

In some embodiments, the releasable connection device is provided both in the tail and at the head of each object-holder container.

If a connection device is provided at both the head and tail of each object-holder container, the activation means and the de-activation means of the connection devices can be staggered at least in height in the treatment chamber.

The releasable connection device and the protruding pin can be positioned crosswise on a head zone and on a tail zone of the same object-holder container, so that the object-holder container can be inserted into the treatment chamber indifferently from the head zone or from the tail zone.

The rotatable arm can comprise, on one side with respect to its axis of rotation, an attachment part able to cooperate with the pin protruding from one side of the second object-holder container.

The wheel, or other rolling element, can be positioned on the other side of the rotatable arm with respect to the side where the attachment portion is positioned.

The rotatable arm can comprise a wheel, or other rolling element, positioned between the axis of rotation and the attachment part.

The wheel positioned between the axis of rotation and the attachment part can be able to engage with at least a first cam positioned on a lateral wall of the treatment chamber and suitable to allow the rotatable arm to pass from the inactive position to the active position.

The wheel positioned between the axis of rotation and the attachment part can be able to cooperate with a second release cam disposed in the lateral wall of the treatment chamber in proximity to the entrance aperture of the chamber and configured to take the rotatable arm from the active position to the inactive position.

The wheel positioned on the other side of the rotatable arm with respect to the side where the attachment part is positioned can be able to cooperate with a third cam disposed in proximity to the entrance aperture of the treatment chamber and suitable to correctly position the rotatable arm in the initial inactive position.

The wheel of the rotatable arm can be able to cooperate with a fourth release cam disposed in the lateral wall of the treatment chamber in proximity to the exit aperture of the chamber and configured to take the rotatable arm from the active position to the inactive position.

The first, second and third cam can be positioned at different heights at least in proximity to the unloading aperture of the object-holder containers.

The invention also concerns a method to connect a plurality of object-holder containers located in sequence in a treatment machine, in particular for washing, thermal disinfection and/or sterilization of objects as defined above.

The method provides: to insert a first object-holder container in a treatment chamber; to insert at least a second object-holder container in the treatment chamber after the first object-holder container has already been inserted in the chamber and to move the object-holder containers toward the inside of the treatment chamber; at least one of the object-holder containers is provided with a releasable connection device automatically activated by activation means disposed in the chamber to reciprocally connect said object-holder containers, so as to allow a simultaneous extraction thereof, acting on the first or last object-holder container inserted. In the present method, the connection device comprises at least one rotatable arm associated with at least one side of a first object-holder container and configured to pass, by means of activation means, from a first inactive release position to a second active attachment position, and also comprises at least one pin protruding from one side of a second object-holder container and suitable to be engaged by the rotatable arm in the active attachment position.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic three-dimensional view of a machine for treating objects , in which an object-holder container is about to be introduced;
- fig. 2 is a schematic front view of a treatment chamber of the machine of fig. 1, in which an object-holder container is about to be introduced;
- fig. 3 is a schematic plan view of a pair of object-holder containers located in sequence, of which a first object-holder container has already been inserted in the treatment chamber;
- figs. 4a-4e are schematic three-dimensional views of a sequence to connect and disconnect the two object-holder containers;
- fig. 5 is a partial three-dimensional view of a machine for treating objects according to the invention described here;

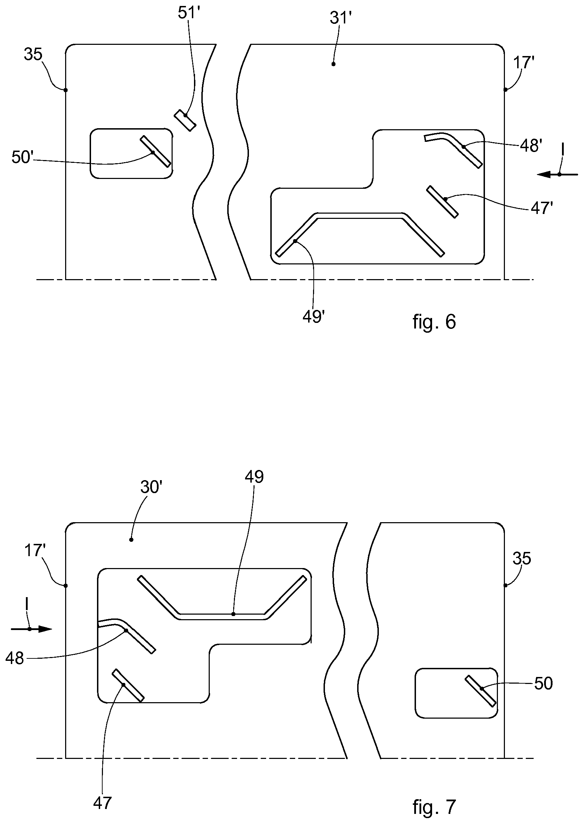

- fig. 6 is a schematic side elevational view of one of the two lateral walls of the treatment chamber;
- fig. 7 is a schematic side elevational view of the other lateral wall of the treatment chamber;
- figs. 8a-8d are schematic side elevational views of some steps of a sequence to attach and release two containers located in sequence operated by a variant of a device for connecting a plurality of object-holder containers according to embodiments described here;
- figs. 9a-9e are three-dimensional schematic views which further illustrate steps for moving the containers using the connection device in figs. 8a-8d.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, a treatment machine 10 comprises at least one treatment chamber 11 in which a plurality of object-holder containers, disposed in sequence, for example, the object-holder containers 12 and 13 can be positioned.

For example, the object-holder containers 12 and 13 can be in the form of racks or suchlike.

Typically, the object-holder containers 12 and 13 comprise a frame 14 provided with at least one head bar 15, see for example the head bar 15 of the object-holder container 13, and at least one tail bar 16, see for example the tail bar 16 of the object-holder container 12.

The bars 15 or 16 are directed in a direction T substantially transverse with respect to a direction L for the insertion and extraction of the object-holder containers 12 and 13 into and from the chamber 11.

The frame 14 also comprises at least one pair of sides 20 and 21, substantially directed in the direction L. The sides 20 and 21 are preferably made at the same height.

The sides 20 and 21 are preferably disposed at the same height as the bars 15 and 16, therefore in proximity to the base of the object-holder containers 12 and 13.

The provision of two or more object-holder containers 12 and 13 disposed in sequence is useful, for example, in the event that the user has problems of bulk for the operations of inserting and extracting the object-holder containers from the treatment machine 10.

In fact, the length of the object-holder container or containers 12 and 13 located in sequence is normally such that the whole length of the treatment chamber 11 is substantially occupied.

Hereafter in the description we will refer, by way of non-restrictive example, to the example shown of two object-holder containers 12 and 13 located in sequence, and to a single-door treatment machine. As can easily be seen, however, the embodiment described here can also concern a pass-through treatment machine, since, in the event of failure, breakdown, error in the cycle or suchlike, as mentioned above, the unloading door is automatically blocked so that, in fact, the loading aperture would also be used to remove the object-holder containers.

The treatment chamber 11 will be provided inside it with means, not shown in the drawings, useful to carry out a specific treatment of the objects located in the object-holder containers 12 and 13, for example washing, thermal disinfection, sterilization or other means.

The chamber 11 of the treatment machine 10, since it is an example of a single-door machine, comprises an aperture 17 for inserting the object-holder containers 12, 13 with the objects to be treated and for extracting the object-holder containers 12, 13 with the treated objects.

The aperture 17 will be opened or closed by means of a corresponding door, not shown in the drawings, which can be driven manually or automatically.

The door for opening and closing the aperture 17 can be a sliding door, for example a rolling shutter door or suchlike, and is able to guarantee an adequate seal once the chamber 11 has been closed to carry out the treatment of the objects located in the object-holder containers 12, 13.

Inside the treatment chamber 11, the object-holder containers 12, 13 rest on a pair of supports 18, for example a pair of parallel tracks.

The object-holder containers 12, 13 can also comprise wheels 19 or suchlike in order to move them inside the chamber 11 in direction L.

On at least one bar of the object-holder container, see for example the head bar 15 of the object-holder container 13, a releasable connection device 22 of the object-holder container 13 is positioned with the object-holder container 12 already inserted in the treatment chamber 11.

In some embodiments, the connection device 22 substantially comprises a sliding block 23 resting on one side of the bar 15 and provided with at least one attachment element 24, protruding from the other side of the bar 15, that is, the external side of the object-holder container 13.

The attachment element 24 can be, for example, shaped like an upside down U and can have an amplitude such as to be able to sit astride at least two adjacent transverse bars of two object-holder containers 12, 13, for example the bars 15 and 16.

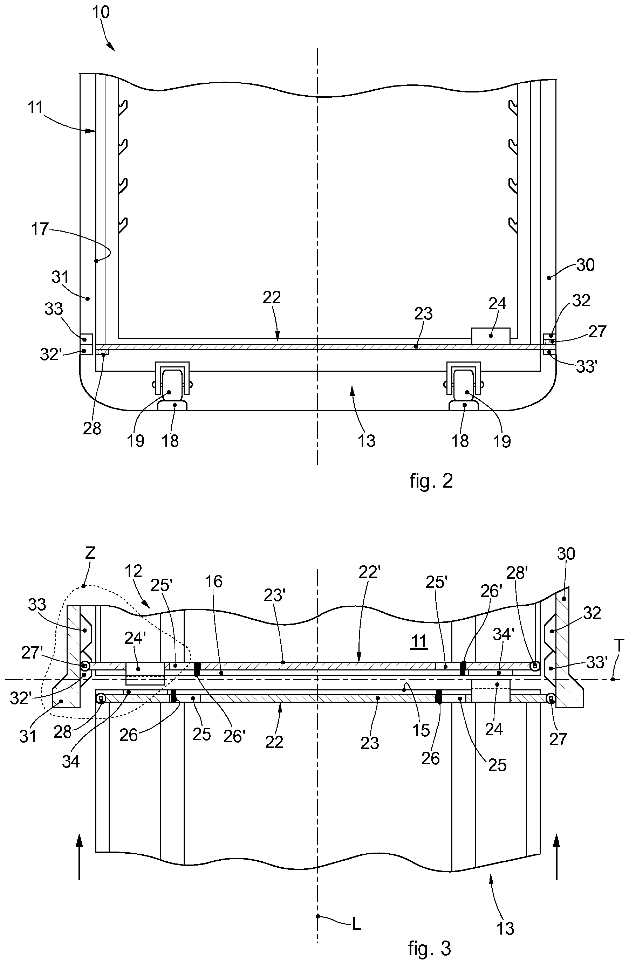

The sliding block 23, see fig. 3, comprises a pair of slits 25, in each of which a pin 26 integral with the bar 15 is inserted. The slits 25 allow the sliding block 23 to slide in direction T, in one sense or the other, with respect to the bar 15. The amplitude of the slits 25 substantially defines the entity of the transverse travel of the sliding block 23.

The sliding block 23 also comprises a rolling element on each end, for example a wheel, see for this purpose the wheels 27 and 28.

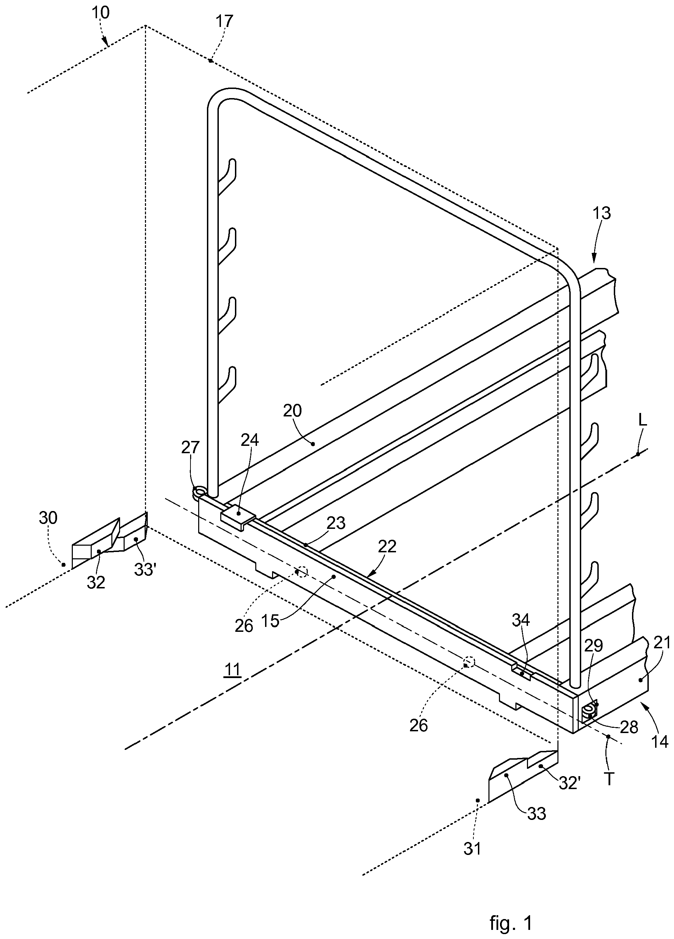

The wheels 27 and 28 are idle and able to protrude alternatively from one side or the other of the object-holder container 13 through suitable holes 29 made in the sides 20 and 21, see for example the hole 29 of fig. 1 made in the side 21 and from which the wheel 28 can exit, as we shall see.

In the example shown, the wheel 27 initially protrudes from the object-holder container 13, while the wheel 28 is housed inside the object-holder container 13.

Preferably, the height at which the wheels 27 and 28 are positioned is different, for example, in the drawings shown, the wheel 27 is situated at a height greater than the wheel 28.

The treatment chamber 11 comprises two opposite lateral walls 30 and 31, on which activation means of the connection device 22 are positioned, for example a cam profile 32 suitably protruding toward the inside of the chamber 11.

The cam profile 32 is positioned at a height such as to cooperate with the wheel 27 protruding from the object-holder container 13, when the object-holder container 13 is inserted in direction L.

Thanks to the cam profile 32, the wheel 27 will be retracted inside the object-holder container 13, determining, as we will see, a transverse activation displacement of the connection device 22 and, in particular, a translation of the sliding block 23 and of the corresponding attachment element 24.

On the lateral wall 31 of the treatment chamber 11 opposite the lateral wall 30, means are positioned to de-activate the connection device 22, for example a cam profile 32', which can be used in the extraction step of the object-holder container. The cam profile 32' also protrudes suitably toward the inside of the chamber 11.

The cam profile 32' that de-activates the connection device 22, and therefore reciprocally separates the object-holder container 12 and the object-holder container 13, is positioned in proximity to the entrance aperture 17 of the chamber 11, so as to take the object-holder container 12, which is more remote and housed more internally in the chamber 11, toward the exit of the chamber 11, hence in an easily grippable position.

The cam profile 32' is positioned at a height such as to cooperate with the wheel 28, which will protrude from the object-holder container 13 when the object-holder container 13 is removed from the chamber 11.

Thanks to the cam profile 32', the wheel 28 will again be retracted inside the object-holder container 13, determining, as we shall see, a transverse de-activation displacement of the connection device 22 and, in particular, a translation of the sliding block 23 and of the corresponding attachment element 24.

The activation cam profile 32, therefore, in this example, is situated at a greater height than the de-activation cam profile 32', but it could also be the other way round.

In the examples shown, in order to guarantee greater uniformity and symmetry in the connection of the object-holder containers 12 and 13 and in order to provide reciprocal connection devices both at the head and tail of the object-holder containers 12 and 13, another connection device 22' is positioned in the tail bar 16 of the object-holder container already inserted in the treatment chamber 11.

The provision of connection devices 22 and 22' at both the head and tail of the object-holder containers 12 and 13 also allows the operator not to confuse the head part with the tail part of the object-holder container 12 or 13, since the head and tail parts will be reciprocally symmetrical.

Structurally, the connection device 22' is very similar to the connection device 22 associated with the bar 15 of the object-holder container 13.

When the connection of the object-holder containers 12 and 13 is complete, preferably, the attachment elements 24 and 24' of the connection devices 22 and 22' will be disposed in specular positions with respect to the longitudinal axis L.

The connection device 22' will therefore be provided with a sliding block 23', at the ends of which corresponding wheels 27' and 28' are positioned.

Since the wheel 27 of the connection device 22 associated with the object-holder container 13 initially protrudes from the object-holder container 13, the wheel 28' of the object-holder container 12, which is on the same side of the wheel 27, will be housed inside the object-holder container 12.

In the same way, since the wheel 27' of the connection device 22' associated with the object-holder container 12 initially protrudes from the object-holder container 12, the wheel 28 of the object-holder container 13, which is on the same side of the wheel 27', will initially be inserted into the object-holder container 13.

The wheel 27' of the connection device 22', which in this example is situated at a greater height than the wheel 28', is therefore able to cooperate with a cam profile 33 to activate the connection device 22'. The cam profile 33 suitably protrudes toward the inside of the chamber 11.

The cam profile 33 is positioned on the lateral wall 31 of the treatment chamber 11 in a longitudinally staggered position with respect to the position of the cam profile 32', so as to allow the alternate drive of the wheels 27' of the object-holder container 12 and of the wheel 28 of the object-holder container 13.

The wheel 28' of the connection device 22' is instead able to cooperate with a cam profile 33' to de-activate the connection device 22'. The cam profile 33' suitably protrudes toward the inside of the chamber 11.

Like the de-activation cam profile 32', the cam profile 33' to de-activate the connection device 22' and thus to separate the object-holder containers 12 and 13 is disposed in proximity to the entrance or exit aperture 17 of the treatment chamber 11.

The cam profile 33' is positioned on the lateral wall 30 of the treatment chamber 11 in a longitudinally staggered position with respect to the position of the cam profile 32, so as to allow the alternate drive of the wheels 28' of the object-holder container 12 and of the wheel 27 of the object-holder container 13.

In the same way as seen for the connection device 22, also the sliding block 23' of the connection device 22' is provided with slits 25' able to be engaged by pins 26' integral with the bar 16, in this case the tail bar of the object-holder container 12.

Substantially therefore, the activation means of the connection devices 22 and 22', comprising for example the cam profiles 32 and 33, are positioned at the same height and in the same longitudinal position in the chamber 11. Similarly, the de-activation means of the connection devices 22 and 22', for example comprising the cam profiles 32' and 33', are also positioned at the same height and in the same longitudinal position in the chamber 11.

The activation cam profiles 32 and 33, as we have seen, are longitudinally staggered with respect to the cam profiles 32' and 33'.

The cam profiles 32 and 33 are also situated at a greater height than the cam profiles 32' and 33'.

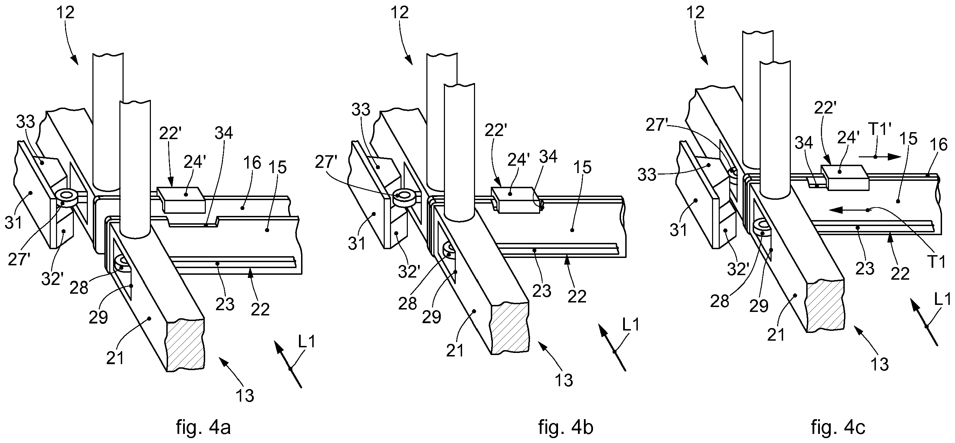

The sequence to connect and disconnect the object-holder containers 12 and 13 shown schematically in figs. 4a-4d substantially relates to the zone Z circled in dashed lines in fig. 3.

Let us suppose that the object-holder container 12 is already inserted in the treatment chamber 11.

The object-holder container 13, for example by means of a rollerway or suchlike, is moved into proximity to the entrance aperture 17 of the treatment chamber 11, as can be seen for example also in figs. 1 or 3.

The rollerway or other approach and removal unit outside the treatment chamber 11 can be provided with movement means with respect to the chamber 11, for example translation means, so as to be able to discharge the object-holder containers 12 and 13 extracted from the chamber 11.

The head bar 15 of the object-holder container 13 is provided with at least a groove 34 of an amplitude such as to allow the temporary passage of the attachment element 24' of the connection device 22' associated with the object-holder container 12.

Similarly, the tail bar 16 of the object-holder container 12 is also provided with a groove 34' of an amplitude such as to allow the temporary passage of the attachment element 24 of the connection device 22 associated with the object-holder container 13.

The object-holder container 13 is thrust in direction L1 inside the treatment chamber 11 until the bar 15 and the bar 16 are reciprocally adjacent, see fig. 4b. As can be seen, the attachment element 24' is positioned above both the bars 15 and 16 and has crossed the groove 34 of the bar 15. Similarly, the attachment element 24 will have passed the groove 34' of the bar 16 and will find itself above both bars 15 and 16.

Continuing in the thrust of the object-holder container 13 inside the treatment chamber 11, the object-holder container 12 will move toward the bottom of the chamber 11.

When the protruding wheel 27' of the connection device 22' meets the cam profile 33, fig. 4c, the connection device 22' and the corresponding attachment element 24' are automatically translated in direction T1', that is to say, in this case, toward the center of the chamber 11. Similarly, when the wheel 27' cooperates with the cam profile 33, the wheel 27 of the connection device 22 cooperates with the cam profile 32, so that the device 22 moves automatically in a direction T1, opposite to the direction T1', thus also toward the center of the chamber 11. In this situation, the wheel 28 of the connection device 22 begins to exit from the hole 29 and to protrude out of the object-holder container 13.

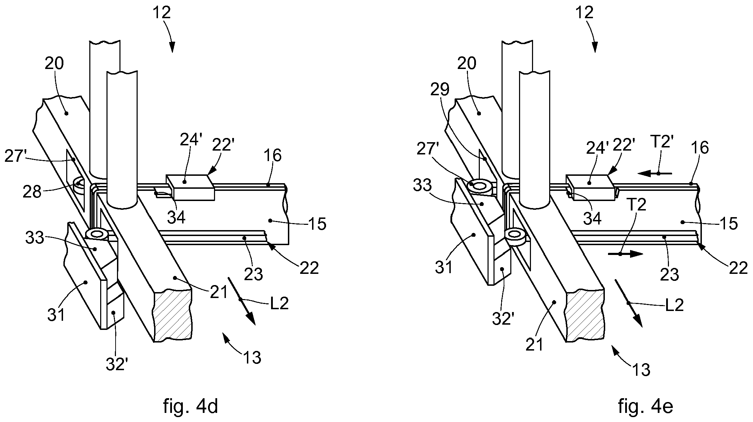

The translation T1 and T1' of the two connection devices 22 and 22' causes the automatic overlapping of the attachment elements 24 and 24' on both the bars 15 and 16, as shown for example in fig. 4d for the attachment element 24'.

When the positioning is completed, as previously mentioned, the attachment elements 24 and 24' will be substantially in a symmetrical position with respect to the longitudinal axis L.

At this point, therefore, the object-holder containers 12 and 13 are attached to each other and the first object-holder container 12 can be taken, always thrusting the object-holder container 13, to the bottom of the treatment chamber 11.

In the extraction step, the object-holder container inserted last, hence the object-holder container 13, is translated in the direction L2, see fig. 4d again, and draws with it the first object-holder container inserted in the chamber 11, that is, the object-holder container 12, since they are reciprocally connected.

Continuing the extraction translation L2, the protruding wheel 28 of the connection device 22 does not interact with the activation cam profile 33, in this case it passes under the cam profile 33 and instead interacts with the de-activation cam profile 32, thus determining an automatic translation in direction T2, see fig. 4e, of the connection device 22.

Similarly, the wheel 28' of the connection device 22' will cooperate with the cam profile 33' so as to automatically retract and translate the connection device 22' in direction T2', and then return the wheel 27' in a position protruding from the object-holder container 12.

The translation T2' will preferably be the same entity and contrary to the translation T1', and the translation T2 will preferably be the same entity and contrary to the translation T1.

The translations T2 and T2' substantially determine the release of the attachment elements 24 and 24' and the return of the object-holder containers 12 and 13 to the situation of fig. 4b, so in this situation the object-holder containers 12 and 13 are again released. The attachment elements 24 and 24' are in fact again in correspondence with the grooves 34 and 34' made on the bars 15 and 16. Continuing in the extraction in direction L2, one passes from the situation of fig. 4b to the situation of fig. 4a.

As mentioned initially, it would also be possible to provide only one connection device 22 or 22', for example imagining that the attachment element 24 or 24' is automatically activated at entrance in order to sit astride both the bars 15 and 16 of the object-holder containers 12 and 13, determining the connection thereof and possibly de-activating automatically at exit, allowing the separation thereof. The automatic activation of the connection device 22 or 22' will be determined, for example, by the cam profile 32 or 33 and its possible automatic de-activation will be determined by the cam profile 32' or 33'.

It is important that the connection device 22 and/or 22' is provided with means which allow its automatic activation, in order to connect the object-holder containers 12 and 13 at the moment of insertion into the chamber 11 and to be able to extract them together once inside the chamber 11. The de-activation of the reciprocal connection of the object-holder containers 12 and 13 can preferably take place automatically and in correspondence with the entrance or exit aperture 17 of the treatment chamber 11, so that the first of the object-holder containers inserted, that is to say, the object-holder container 12 located farthest down the chamber 11, at the moment of separation from the second object-holder container 13 is in proximity to the entrance aperture 17 of the chamber 11. This separation could however also take place manually, after the extraction of the object-holder containers 12 and 13.

The activation means of the connection devices 22 and/or 22', and hence the reciprocal connection of the object-holder containers 12 and 13, for example the cam profiles 32 and 33, could also be disposed in more internal positions along the lateral walls 30 and 31 of the treatment chamber 11, provided, substantially, they are within a distance smaller than the travel of the object-holder containers 12 and 13 in the treatment chamber 11.

In fact, in the insertion step, the object-holder containers 12 and 13 do not need to be reciprocally joined, the reciprocal connection between them is necessary in the extraction step, when pulling the outermost object-holder container it must also be possible to pull out the innermost container.

The present treatment machine 10 is therefore equipped with one or more connection devices 22 and 22' and with activation and possible de-activation means, that is, for example the cam profiles 32, 32', 33, 33', of an essentially mechanical type, therefore able to operate even in onerous and difficult operating conditions, such as those of a sterilization machine for example where the working temperatures can also be quite high, even up to 140°C and where, therefore, it is inadvisable to use motorized and/or electric or electronic means for the reciprocal and automatic connection of the object-holder containers inside the chamber.

As previously mentioned, moreover, the object-holder containers 12 and 13 can be disposed outside the chamber on a rollerway or other guide and support system to be inserted into and removed from the chamber 11. As we said, the rollerway or other guide system could be mobile with respect to the treatment chamber 11, so as to be able to be displaced for example, in order to move the object-holder containers 12 and 13 located on the rollerway to a zone different from that in which the treatment chamber 11 is located.

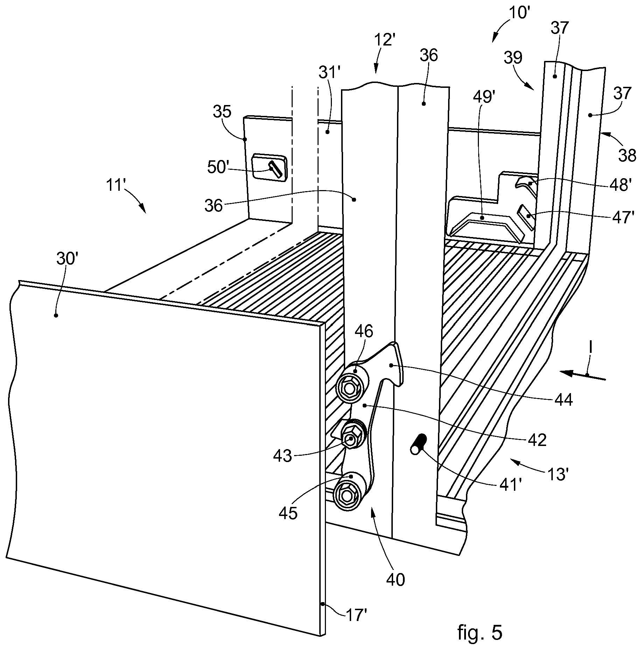

Fig. 5 shows a preferred embodiment of the present treatment machine 10'.

The treatment machine 10' can be a pass-through machine for example, provided with a treatment chamber 11' in which the loading aperture 17' is provided for the object-holder containers 12' and 13' and an aperture 35 to unload the object-holder containers 12' and 13'.

The unloading aperture 35 is located on the opposite side of the treatment chamber 11' with respect to the loading aperture 17.

The object-holder containers 12' and 13', in normal functioning situations, can be loaded from the aperture 17 in direction I and unloaded from the aperture 35, again in direction I.

Each of the object-holder containers 12' and 13' comprises opposite sides 36 and 37, which can for example be defined by uprights situated in a head zone 38 or in a tail zone 39 of each object-holder container 12' and 13'.

The head zone 38 of one object-holder container, for example the object-holder container 13' in fig. 5, will come into contact with the tail zone 39 of an object-holder container previously inserted into the treatment chamber 11', for example the object-holder container 12'.

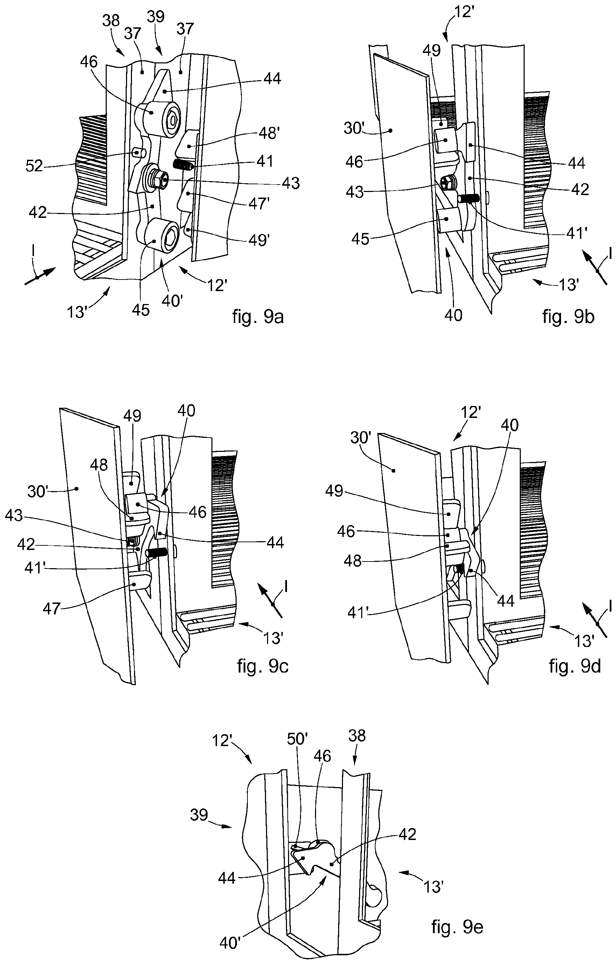

On one side of the tail zone 39 of the object-holder container 12', for example side 36, it is provided to apply a releasable connection device 40, while on the opposite side, that is, side 37, a pin 41 is provided, protruding toward the outside, see for example fig. 9a.

The object-holder container 13' will comprise, on one side of the head zone 38, in this case side 36, a protruding pin 41' suitable to engage with the releasable connection device 40 provided in the object-holder container 12'.

The object-holder container 13' will comprise, on the side 37 of the head zone 38 and outside the same, a corresponding releasable connection device 40' able to cooperate with the pin 41 protruding from the side 37 of the tail zone 39 of the object-holder container 12', see for example fig. 9a.

In substance, the object-holder containers 12' or 13' are provided, in their respective head and tail zones, with a releasable connection device 40, 40' on at least one side and a pin 41, 41' protruding on the opposite side.

The releasable connection device 40, 40' and the protruding pin 41, 41' are preferably positioned in a crosswise manner on the head zone 38 and on the tail zone 39 of a same object-holder container 12' or 13', so that the object-holder container 12' or 13' can be inserted into the treatment chamber 11' indifferently from the head zone 38 or from the tail zone 39.

For example, an object-holder container 12' or 13' can provide the releasable connection device 40, 40' on the left side of the tail zone 39 and the pin 41, 41' protruding on the right side of the tail zone. In this case, in the head zone 38 of the object-holder container 12' or 13', the releasable connection device 40, 40' will be positioned on the right side, while the protruding pin 41, 41' will be positioned on the left side.

According to the invention, the releasable connection device, see for example device 40, comprises a rotatable arm 42, which is connected by means of a corresponding rotation pin 43, for example, on the side 36 of the object-holder container 12', as described using fig. 5.

The pin 43 therefore represents the axis of rotation around which the rotatable arm 42 can oscillate.

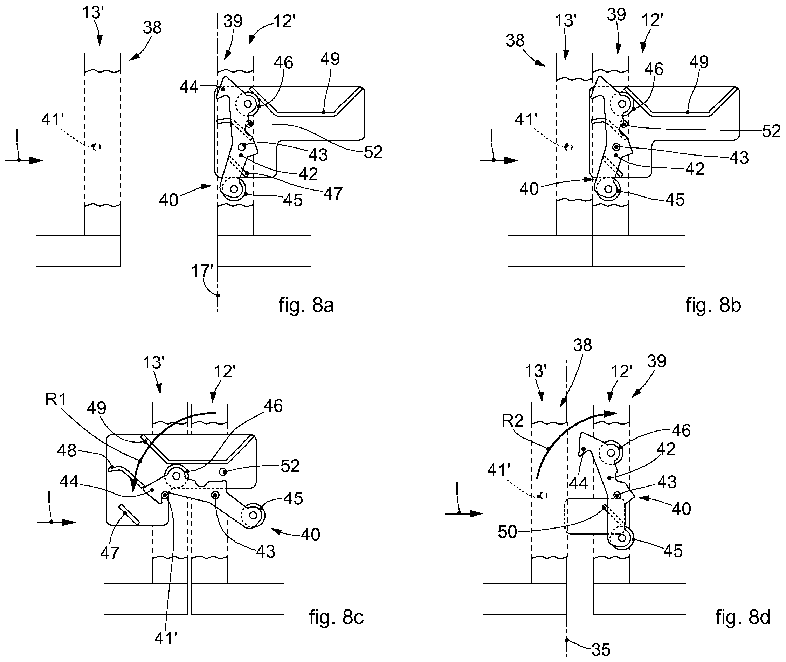

The rotatable arm 42, substantially, provides an inactive position, in which it can be essentially in the vertical direction, as described with reference to fig. 5, and an active position, in which it can be substantially in a horizontal position, as for example in fig. 8c.

The rotation pin 43 can be located in a substantially central position with respect to the development of the rotatable arm 42.

With respect to the rotation pin or axis 43, the rotatable arm 42 comprises, on one side, an attachment part 44, able to cooperate with the pin 41'.

The rotatable arm 42 comprises, on the other side with respect to said attachment part 44, a wheel 45, or other rolling element, the function of which will be described below.

Between the rotation pin 43 and the attachment part 44, the rotatable arm 42 can provide a wheel 46, or other rolling element, the function of which will be described below.

The rotation pin 43 is attached, preferably removably, to the side 36 of the object-holder container 12' and can optionally provide cup-shaped or similar elastic means so as to provide the rotatable arm 42 with a certain friction and therefore so as to stably maintain the rotatable arm 42 in the various positions in which it is located during functioning, as we will see below.

The releasable connection device 40, 40' can be activated by releasable connection means, for example a series of cams disposed on the internal surface of the lateral walls 30' and 31' of the treatment chamber 11'.

In proximity to the entrance side of the treatment chamber 11', hence the side of the aperture 17', the lateral walls 30' and 31' comprise a series of cams 47, 48, 49 and 47', 48', 49'.

The cams 47, 48 and 49 situated on the lateral wall 30' are disposed in sequence and each at a certain height on the lateral wall 30', for example a first cam 49 is provided, situated at a greater height, a second cam 48 situated at an intermediate height and a third cam 47 situated at a greater height.

The cams 47', 48' and 49' situated on the lateral wall can be disposed as follows: the first cam 49' is situated at a greater height and is substantially specular to the first cam 49; the third cam 47' is situated at an intermediate height and the second cam 48' is situated at a greater height.

On the exit side of the treatment chamber 11', that is, the side of the aperture 35, the lateral wall 30' comprises at least another cam 50.

The lateral wall 31' also comprises at least one fourth cam 50' in proximity to the exit aperture.

Optionally, the lateral wall 31' could also provide another fifth cam 51'.

The fifth cam 51' could also be provided on the lateral wall 30' of the treatment chamber in proximity to the exit aperture 35, similar to that provided for the lateral wall 31'.

An example of activation and possible de-activation of the releasable connection device 40, 40' of the object-holder containers 12' and 13' will be clear from the following description and by observing figs. 8a-8d and figs. 9a-9d.

Fig. 8a shows the object-holder container 12' inserted in the treatment chamber 11' with its tail zone 39 in proximity to the aperture 17' of the treatment chamber 11'.

The releasable connection device 40 of the object-holder container 12' is located correctly in a substantially vertical position, with the wheel 46 which is in contact with an initial portion of the first cam 49.

If the object-holder container 12' had been inserted with the rotatable arm 42 of the device 40 in a different position from that shown in fig. 8a, the third cam 47, cooperating with the first wheel 45, would have returned the rotatable arm 42 to the position of fig. 8a, thanks to its proper rotation around the pin 43.

The correct and substantially vertical position of the releasable connection device 40 is also guaranteed by an abutment element 52 made on the side of the object-holder container 12', for example side 36.

By thrusting the object-holder container 13', the head zone 38 of the object-holder container 13' is taken into contact with the tail zone 39 of the object-holder container 12', see fig. 8b.

By thrusting the object-holder container 13' further, the rotatable arm 42 of the releasable connection device 40 is rotated in the direction of the arrow R1 around the pin 43, thanks to the sliding of the wheel 46 along the first cam 49.

The rotation of the rotatable arm 42 around the pin 43 determines the engagement of the attachment part 44 on the pin 41' of the object-holder container 13', so that the object-holder containers 12' and 13' are advantageously attached to each other, see fig. 8c.

The attachment sequence just described is also shown in 3D in figs. 9b, 9c and 9d.

The first cam 49, therefore, represents an example of activation means of the rotatable arm 42, that is, the passage from the inactive release position to the active attachment position.

Similarly, the releasable connection device 40' of the object-holder container 13', thanks to the action of the first cam 49', will engage with the pin 41 protruding from the object-holder container 12'.

The object-holder containers 12' and 13' are then taken to the correct position in the treatment chamber 11', where the objects contained therein are treated in the necessary manner and for the necessary times, provided by the treatment to which they are to be subjected.

If the washing cycle proceeds regularly, in the case of a pass-through machine 10', for example, the object-holder containers 12' and 13' will be extracted from the unloading side of the machine 10', that is, the aperture 35, then the object-holder container 12', which had been inserted first on the loading side, is pulled and this operation also allows the simultaneous extraction of the object-holder container 13', since the object-holder containers 12' and 13', as can be seen, are attached thanks to the releasable connection devices 40, 40'.

Fig. 8d shows schematically an automatic release step of the object-holder containers 12' and 13' from the unloading side, that is, the aperture 35: the fourth cam 50, in this case, engages with the first wheel 45 of the rotatable arm 42 so as to rotate the rotatable arm 42 around the pin 43 in direction R2, opposite to direction R1.

The rotation R2 thus allows to make the rotatable arm 42 pass from the substantially horizontal attachment position of fig. 8c to the substantially vertical position of fig. 8d, that is, where it is released from the pin 41'. In this step, by pulling the object-holder container 12', the object-holder container 13' will remain with its head zone 38 in proximity to the aperture 35.

Similarly, the releasable connection device 40' will also move into the separation position thanks to the fourth cam 50' on which the wheel 46 of the releasable connection device 40' will engage, see also fig. 9e.

The fifth cam 51' has the function of positioning the releasable connection device 40' in a substantially horizontal position, if the object-holder container 13' is returned from the exit side toward the entrance side, that is, toward the aperture 17', so as not to interfere with the cams 47', 48' and 49'.

As we said, if it were necessary to return the object-holder containers 12' and 13', already attached to each other and disposed in the treatment chamber 11', and make them exit from the same loading side from which they entered, that is, the side of the aperture 17, in this case the last inserted object-holder container is pulled, that is, the object-holder container 13', in the opposite direction to direction I.

In this situation the second cam 48 can come into play which, engaging with the wheel 46, allows to take the releasable connection device 40 from the substantially horizontal position to the substantially vertical release position. For example, consider fig. 8c and imagine moving the object-holder containers 12' and 13' in the opposite direction to direction I: at a certain point the wheel 46 will engage the second cam 48 and the rotatable arm 42 will rotate around the pin 43 in the opposite direction to direction R1 as shown, so that the attachment portion 44 disengages from the pin 41'.

Similarly, in such a situation, the connection device 40' will be disposed in such a way that its rotatable arm 42 rotates around the corresponding rotation pin so as to disengage from the pin 41.

The object-holder containers 12', 13' used in the present machine 10', in some applications where for example it is required to support heavy objects or a large quantity of objects to be treated, can include a frame formed by elements of a certain thickness, such as bars or other, suitable to support said objects. The object-holder containers themselves, therefore, although being able to slide on guides, wheels or suchlike, inside and outside the treatment chamber 11', must be provided with a certain resistance and can themselves be endowed with a certain weight. The releasable connection devices 40, 40', associable with the pins 41, 41', have proved particularly effective and advantageous for these types of object-holder containers with a resistant structure and in some cases endowed with a certain weight.

As an alternative to what is shown here, the activation means represented for example by the cam profiles 32, 32' and/or 33, 33' could be positioned on a structure inside the chamber 11 and which acts as a support for the sliding of the object-holder container 12, 13, for example on the supports 18, instead of on the lateral walls 30 and 31 of the chamber 11. Similarly, the cams 48, 48' and 49, 49' could also be positioned on a support structure which allows the sliding of the object-holder containers 12', 13', instead of being positioned on the lateral walls 30', 31'.

It is also clear that, although the present invention has been described with reference to some specific examples, a person of skill in the art shall certainly be able to achieve many other equivalent forms of machine for treating objects, in particular for washing, thermal disinfection and/or sterilization of objects, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Machine for treating objects, in particular washing, thermal disinfection and/or sterilization of objects, comprising:
at least a treatment chamber (11') comprising an entrance aperture (17) and lateral walls (30', 31'), the treatment chamber being configured to house a plurality of object-holder containers (12', 13') located in sequence;
a first object-holder container (12') comprising opposite sides (36, 37) and at least a releasable connection device (40, 40') comprising: at least one rotatable arm (42) provided on at least one of said opposite sides (36, 37) of said first object-holder container (12') and configured to pass, by means of activation means (32, 33, 49, 49') positioned in the treatment chamber (11'), from a first inactive release position to a second active attachment position; and wheel or other rolling element (45, 46),
said machine further comprising a second object-holder container (13') comprising opposite sides (36, 37) and at least one pin (41') protruding from one side (36, 37) of said second object-holder container (13') and suitable to be engaged by said rotatable arm (42) in the second active attachment position,
said activation means positioned in the treatment chamber comprising at least a cam (49, 49') disposed on the internal surface of the lateral walls (30', 31') of the treatment chamber (11'),
whereby said releasable connection device (40, 40'), said pin (41') and said activation means (49, 49') are configured to cooperate, so that, inserting the second object-holder container (13') in the treatment chamber (11') after at least a first object-holder container (12') already inserted in said chamber (11') and moving said second object-holder containers (13') toward the inside of the chamber (11'), said releasable connection device (40, 40') is automatically activated by said activation means (49, 49'), by thrusting said second object-holder container (13') against said first object-holder container (12'), causing the rotation of said rotatable arm (42) from said first inactive release position to said second active attachment position thanks to the sliding of said wheel or other rolling element (45) along said cam (49, 49'), in order to reciprocally connect said object-holder containers (12' 13'), so as to allow a simultaneous extraction thereof, acting on the first or last object-holder container (12', 13') inserted.

2. Machine as in claim 1, **characterized in that** it comprises de-activation means (50, 50') of said releasable connection device (40, 40') so as to automatically separate said object-holder containers (12', 13') during an extraction step of the object-holder containers (12', 13') from the chamber (11').

3. Machine as in any claim hereinbefore, **characterized in that** said releasable connection device (40, 40') is provided in both a tail zone (39) and a head zone (38) of each object-holder container (12', 13').

4. Machine as in claim 1, **characterized in that** said releasable connection device (40, 40') and said protruding pin (41, 41') are positioned in a crosswise manner on a head zone (38) and on a tail zone (39) of a same object-holder container (12', 13'), so that said object-holder container (12', 13') can be inserted into the treatment chamber (11') indifferently from the head zone (38) or from the tail zone (39).

5. Machine as in any of the claims hereinbefore, **characterized in that** said rotatable arm (42) comprises, on one side with respect to its axis of rotation (43), an attachment part (44) able to cooperate with the pin (41, 41') protruding from one side (36, 37) of said second object-holder container (12, 13').

6. Machine as in claim 5, **characterized in that** said wheel (45), or other rolling element, is positioned on the other side of said rotatable arm (42) with respect to the side where the attachment part (44) is positioned.

7. Machine as in claim 5, **characterized in that** said rotatable arm (42) comprises a wheel (46), or other rolling element, positioned between said axis of rotation (43) and said attachment part (44).

8. Machine as in claim 7, **characterized in that** said wheel (46) is able to engage with at least one first cam (49, 49') positioned on a lateral wall (30', 31') of the chamber (11') and suitable to allow the passage of said rotatable arm (42) from the inactive position to the active position.

9. Machine as in claim 7, **characterized in that** said wheel (46) of the rotatable arm (42) is able to cooperate with a second release cam (48, 48') disposed in the lateral wall (30', 31') of the treatment chamber (11') in proximity to the entrance aperture (17') of said chamber (11') and configured to take the rotatable arm (42) from the active position to the inactive position.

10. Machine as in claim 6, **characterized in that** said wheel (45) is able to cooperate with a third cam (47, 47') disposed in proximity to the entrance aperture (17') of the treatment chamber (11') and suitable to correctly position the rotatable arm (42) in the initial inactive position.

11. Machine as in claim 6 or 7, **characterized in that** said wheel (46, 45) of the rotatable arm (42) is able to cooperate with a fourth release cam (50, 50') disposed in the lateral wall (30, 30') of the treatment chamber (11') in proximity to the exit aperture (35) of said chamber (11') and configured to take the rotatable arm (42) from the active position to the inactive position.

12. Machine as in claims 8, 9 and 10, **characterized in that** said first cam (49, 49'), said second cam (48, 48') and said third cam (47, 47') are positioned at different heights at least in proximity to the aperture (35) for loading the object-holder containers (12', 13').

13. Method to connect a plurality of object-holder containers located in sequence in a treatment machine, in particular for washing, thermal disinfection and/or sterilization of objects as in any of the claims hereinbefore, wherein said method provides: to insert a first object-holder container (12') in a treatment chamber (11'); to insert at least a second object-holder container (13') in the treatment chamber (11') after said first object-holder container (12') which has already been inserted in said treatment chamber (11'), and to move said object-holder containers (12', 13') toward the inside of the treatment chamber (11'); at least one of said object-holder containers (12') being provided with a releasable connection device (40, 40') automatically activated by activation means (49, 49') disposed in the treatment chamber (11') to reciprocally connect said object-holder containers (12', 13'), so as to allow a simultaneous extraction thereof, acting on the first or last object-holder container (12', 13') inserted, said releasable connection device (40, 40') comprising at least one rotatable arm (42) associated with at least one side (36, 37) of a first object-holder container (12') and configured to pass, by means of said activation means (49, 49'), from a first inactive release position to a second active attachment position, and also comprising at least one pin (41') protruding from one side (36, 37) of a second object-holder container (13') and suitable to be engaged by said rotatable arm (42) in the active attachment position.

## Patentansprüche

1. Maschine zur Behandlung von Objekten, insbesondere zum Waschen, zur thermischen Desinfektion und/oder zum Sterilisieren von Objekten, umfassend:
zumindest eine Behandlungskammer (11') umfassend eine Eingangsöffnung (17) und Seitenwände (30', 31'), wobei die Behandlungskammer dafür ausgelegt ist, eine Vielzahl von Objekthalterbehältern (12', 13') aufzunehmen, die nacheinander angeordnet sind;
einen ersten Objekthalterbehälter (12'), der gegenüberliegende Seiten (36, 37) und zumindest eine lösbare Verbindungsvorrichtung (40, 40') umfasst, welche umfasst: zumindest einen drehbaren Arm (42), der an zumindest einer der gegenüberliegenden Seiten (36, 37) des ersten Objekthalterbehälters (12') vorgesehen ist und dafür ausgelegt ist, durch Betätigungsmittel (32, 33, 49, 49'), die in der Behandlungskammer (11') positioniert sind, von einer ersten inaktiven Auslösestellung zu einer zweiten aktiven Befestigungsstellung überzugehen; und ein Rad oder sonstiges rollendes Element (45, 46),
wobei die Maschine ferner einen zweiten Objekthalterbehälter (13') umfasst, der gegenüberliegende Seiten (36, 37) und zumindest einen Stift (41') umfasst, der aus einer Seite (36, 37) des zweiten Objekthalterbehälters (13') vorspringt und dafür geeignet ist, vom drehbaren Arm (42) in der zweiten aktiven Befestigungsstellung eingegriffen zu werden,
wobei die Betätigungsmittel, die in der Behandlungskammer positioniert sind, zumindest einen Nocken (49, 49') umfassen, der auf der Innenfläche der Seitenwände (30', 31') der Behandlungskammer (11') angeordnet ist,
wobei die lösbare Verbindungsvorrichtung (40, 40'), der Stift (41') und die Betätigungsmittel (49, 49') dafür ausgelegt sind, miteinander zusammenzuwirken, so dass, durch Einfügen des zweiten Objekthalterbehälters (13') in die Behandlungskammer (11') nach zumindest einem ersten Objekthalterbehälter (12'), der in der Kammer (11') bereits eingefügt ist, und durch Bewegen der zweiten Objekthalterbehälter (13') zum Inneren der Kammer (11') hin, die lösbare Verbindungsvorrichtung (40, 40') von den Betätigungsmitteln (49, 49') dadurch automatisch betätigt wird, dass der zweite Objekthalterbehälter (13') gegen den ersten Objekthalterbehälter (12') geschoben wird, wodurch die Drehung des drehbaren Arms (42) von der ersten inaktiven Auslösestellung zur zweiten aktiven Befestigungsstellung aufgrund des Gleitens des Rads oder sonstigen rollenden Elementes (45) entlang des Nockens (49, 49') bewirkt wird, um die Objekthalterbehälter (12', 13') wechselseitig zu verbinden, so dass ein gleichzeitiges Herausziehen derselben ermöglicht wird, indem auf den ersten oder auf den letzten eingeführten Objekthalterbehälter (12', 13') eingewirkt wird.

2. Maschine nach Anspruch 1, **dadurch gekennzeichnet, dass** sie Abschaltmittel (50, 50') der lösbaren Verbindungsvorrichtung (40, 40') umfasst, so dass die Objekthalterbehälter (12', 13') während eines Herauszieheschrittes der Objekthalterbehälter (12', 13') aus der Kammer (11') automatisch getrennt werden.

3. Maschine nach einem der vorhergehenden Ansprüche, **dadurch gekennzeichnet, dass** die lösbare Verbindungsvorrichtung (40, 40') sowohl an einem Endbereich (39) als auch an einem Kopfbereich (38) jedes Objekthalterbehälters (12', 13') vorgesehen ist.

4. Maschine nach Anspruch 1, **dadurch gekennzeichnet, dass** die lösbare Verbindungsvorrichtung (40, 40') und der vorspringende Stift (41, 41') kreuzweise an einem Kopfbereich (38) und an einem Endbereich (39) desselben Objekthalterbehälters (12', 13') positioniert sind, so dass der Objekthalterbehälter (12', 13') in die Behandlungskammer (11') hinein ohne Unterschied entweder vom Kopfbereich (38) oder vom Endbereich (39) eingeführt werden kann.

5. Maschine nach einem der vorhergehenden Ansprüche, **dadurch gekennzeichnet, dass** der drehbare Arm (42), an einer Seite im Hinblick auf seine Drehachse (43), einen Befestigungsteil (44) umfasst, der mit dem Stift (41, 41') zusammenarbeiten kann, der aus einer Seite (36, 37) des zweiten Objekthalterbehälters (12, 13') vorspringt.

6. Maschine nach Anspruch 5, **dadurch gekennzeichnet, dass** das Rad (45) oder sonstige rollende Element an der anderen Seite des drehbaren Arms (42) im Hinblick auf die Seite positioniert ist, an der der Befestigungsteil (44) positioniert ist.

7. Maschine nach Anspruch 5, **dadurch gekennzeichnet, dass** der drehbare Arm (42) ein Rad (46), oder sonstiges rollendes Element, umfasst, das zwischen der Drehachse (43) und dem Befestigungsteil (44) positioniert ist.

8. Maschine nach Anspruch 7, **dadurch gekennzeichnet, dass** das Rad (46) mit zumindest einem ersten Nocken (49, 49') eingreifen kann, der an einer Seitenwand (30', 31') der Kammer (11') positioniert ist und dazu geeignet ist, den Übergang des drehbaren Armes (42) von der inaktiven Stellung zur aktiven Stellung zu ermöglichen.

9. Maschine nach Anspruch 7, **dadurch gekennzeichnet, dass** das Rad (46) des drehbaren Armes (42) mit einem zweiten Auslösenocken (48, 48') zusammenwirken kann, der in der Seitenwand (30', 31') der Behandlungskammer (11') in der Nähe der Eingangsöffnung (17') der Kammer (11') angeordnet ist und dafür ausgelegt ist, den drehbaren Arm (42) von der aktiven Stellung zur inaktiven Stellung zu nehmen.

10. Maschine nach Anspruch 6, **dadurch gekennzeichnet, dass** das Rad (45) mit einem dritten Nocken (47, 47') zusammenwirken kann, der in der Nähe der Eingangsöffnung (17') der Behandlungskammer (11') angeordnet ist und dazu geeignet ist, den drehbaren Arm (42) in der anfänglichen inaktiven Stellung richtig zu positionieren.

11. Maschine nach Anspruch 6 oder 7, **dadurch gekennzeichnet, dass** das Rad (46, 45) des drehbaren Armes (42) mit einem vierten Auslösenocken (50, 50') zusammenwirken kann, der in der Seitenwand (30, 30') der Behandlungskammer (11') in der Nähe der Ausgangsöffnung (35) der Kammer (11') angeordnet ist und dafür ausgelegt ist, den drehbaren Arm (42) von der aktiven Stellung zur inaktiven Stellung zu nehmen.

12. Maschine nach den Ansprüchen 8, 9 und 10, **dadurch gekennzeichnet, dass** der erste Nocken (49, 49'), der zweite Nocken (48, 48') und der dritte Nocken (47, 47') bei verschiedenen Höhen zumindest in der Nähe der Öffnung (35) zur Beladung der Objekthalterbehälter (12', 13') positioniert sind.

13. Verfahren zum Verbinden einer Vielzahl von Objekthalterbehältern, die nacheinander in einer Behandlungsmaschine angeordnet sind, insbesondere zum Waschen, zur thermischen Desinfektion und/oder zum Sterilisieren von Objekten nach einem der vorhergehenden Ansprüche, worin das Verfahren vorsieht: einen ersten Objekthalterbehälter (12') in eine Behandlungskammer (11') einzufügen; zumindest einen zweiten Objekthalterbehälter (13') in die Behandlungskammer (11') nach dem ersten Objekthalterbehälter (12'), der in die Behandlungskammer (11') bereits eingefügt worden ist, einzufügen und die Objekthalterbehälter (12', 13') zum Inneren der Behandlungskammer (11') hin zu bewegen; wobei zumindest einer der Objekthalterbehälter (12') mit einer lösbaren Verbindungsvorrichtung (40, 40') versehen ist, die durch Betätigungsmittel (49, 49') automatisch betätigt wird, die in der Behandlungskammer (11') angeordnet sind, um die Objekthalterbehälter (12', 13') wechselseitig zu verbinden, so dass ein gleichzeitiges Herausziehen derselben ermöglicht wird, indem auf den ersten oder auf den letzten eingeführten Objekthalterbehälter (12', 13') eingewirkt wird, wobei die lösbare Verbindungsvorrichtung (40, 40') zumindest einen drehbaren Arm (42) umfasst, der zumindest einer Seite (36, 37) eines ersten Objekthalterbehälters (12') zugeordnet ist und dafür ausgelegt ist, durch die genannten Betätigungsmittel (49, 49'), von einer ersten inaktiven Auslösestellung zu einer zweiten aktiven Befestigungsstellung überzugehen, und auch umfassend zumindest einen Stift (41'), der aus einer Seite (36, 37) eines zweiten Objekthalterbehälters (13') vorspringt und dafür geeignet ist, vom drehbaren Arm (42) in der aktiven Befestigungsstellung eingegriffen zu werden.

## Revendications

1. Machine de traitement d'objets, en particulier de lavage, de désinfection thermique et / ou de stérilisation d'objets, comprenant :
au moins une chambre de traitement (11') comprenant une ouverture d'entrée (17) et des parois latérales (30', 31'), la chambre de traitement étant configurée pour loger une pluralité de récipients porte-objets (12', 13') situés en succession ;
un premier récipient porte-objet (12') comprenant des côtés opposés (36, 37) et au moins un dispositif de connexion libérable (40, 40') comprenant : au moins un bras rotatif (42) prévu sur au moins un desdits côtés opposés (36, 37) dudit premier récipient porte-objet (12') et configuré pour passer, au moyen de moyens d'activation (32, 33, 49, 49') positionnés dans la chambre de traitement (11'), d'une première position de libération inactive à une deuxième position de fixation active ; et une roue ou autre élément roulant (45, 46),
ladite machine comprenant en outre un deuxième récipient porte-objet (13') comprenant des côtés opposés (36, 37) et au moins un ergot (41') faisant saillie d'un côté (36, 37) dudit deuxième récipient porte-objet (13') et apte à être en relation d'engagement avec ledit bras rotatif (42) dans la deuxième position de fixation active,
lesdits moyens d'activation positionnés dans la chambre de traitement comprenant au moins une came (49, 49') disposé sur la surface interne des parois latérales (30', 31') de la chambre de traitement (11'),
de sorte que ledit dispositif de connexion libérable (40, 40'), ledit ergot (41') et lesdits moyens d'activation (49, 49') sont configurés pour coopérer, de sorte que, par l'insertion du deuxième récipient porte-objet (13') dans la chambre de traitement (11') après au moins un premier récipient porte-objet (12') déjà inséré dans ladite chambre (11') et par le déplacement desdits deuxièmes récipients porte-objets (13') vers l'intérieur de la chambre (11'), ledit dispositif de connexion libérable (40, 40') soit automatiquement activé par lesdits moyens d'activation (49, 49'), en poussant ledit deuxième récipient porte-objets (13') contre ledit premier récipient porte-objets (12'), provoquant la rotation dudit bras rotatif (42) de ladite première position de libération inactive à ladite deuxième position de fixation active grâce au coulissement de ladite roue ou autre élément roulant (45) le long de ladite came (49, 49'), afin de connecter mutuellement lesdits récipients porte-objets (12' 13'), de manière à permettre une extraction simultanée de ceux-ci, agissant sur le premier ou le dernier récipient porte-objet (12', 13') inséré.

2. Machine selon la revendication 1, **caractérisée en ce qu'**elle comprend des moyens de désactivation (50, 50') dudit dispositif de connexion libérable (40, 40') de manière à séparer automatiquement lesdits récipients porte-objets (12', 13') lors d'une étape d'extraction des récipients porte-objets (12', 13') hors de la chambre (11').

3. Machine selon l'une quelconque des revendications précédentes, **caractérisée en ce que** ledit dispositif de connexion libérable (40, 40') est prévu à la fois dans une zone postérieure (39) et une zone antérieure (38) de chaque récipient porte-objet (12', 13').

4. Machine selon la revendication 1, **caractérisée en ce que** ledit dispositif de connexion libérable (40, 40') et ledit ergot saillant (41, 41') sont positionnés d'une manière transversale sur une zone antérieure (38) et sur une zone postérieure (39) d'un même récipient porte-objet (12', 13'), de sorte que ledit récipient porte-objet (12', 13') est apte à être inséré dans la chambre de traitement (11') indifféremment par la zone antérieure (38) ou par la zone arrière (39).

5. Machine selon l'une quelconque des revendications précédentes, **caractérisée en ce que** ledit bras rotatif (42) comprend, d'un côté par rapport à son axe de rotation (43), une partie d'accrochage (44) apte à coopérer avec l'ergot (41, 41') faisant saillie d'un côté (36, 37) dudit deuxième récipient porte-objet (12, 13').

6. Machine selon la revendication 5, **caractérisée en ce que** ladite roue (45), ou autre élément roulant, est positionnée de l'autre côté dudit bras rotatif (42) par rapport au côté au niveau duquel la partie d'accrochage (44) est positionnée.

7. Machine selon la revendication 5, **caractérisée en ce que** ledit bras rotatif (42) comprend une roue (46), ou autre élément roulant, positionnée entre ledit axe de rotation (43) et ladite partie d'accrochage (44).

8. Machine selon la revendication 7, **caractérisée en ce que** ladite roue (46) est apte à venir en engagement avec au moins une première came (49, 49') positionnée sur une paroi latérale (30', 31') de la chambre (11') et adaptée pour permettre le passage dudit bras rotatif (42) de la position inactive à la position active.

9. Machine selon la revendication 7, **caractérisée en ce que** ladite roue (46) du bras rotatif (42) est apte à coopérer avec une deuxième came de libération (48, 48') disposée dans la paroi latérale (30', 31') de la chambre de traitement (11') à proximité de l'ouverture d'entrée (17') de ladite chambre (11') et configuré pour amener le bras rotatif (42) de la position active à la position inactive.

10. Machine selon la revendication 6, **caractérisée en ce que** ladite roue (45) est apte à coopérer avec une troisième came (47, 47') disposée à proximité de l'ouverture d'entrée (17') de la chambre de traitement (11') et adaptée pour positionner correctement le bras rotatif (42) dans la position inactive initiale.

11. Machine selon la revendication 6 ou la revendication 7, **caractérisée en ce que** ladite roue (46, 45) du bras rotatif (42) est apte à coopérer avec une quatrième came de libération (50, 50') disposée dans la paroi latérale (30, 30') de la chambre de traitement (11') à proximité de l'ouverture de sortie (35) de ladite chambre (11') et configurée pour amener le bras rotatif (42) de la position active à la position inactive.

12. Machine selon les revendications 8, 9 et 10, **caractérisée en ce que** ladite première came (49, 49'), ladite deuxième came (48, 48') et ladite troisième came (47, 47') sont positionnées à différentes hauteurs au moins à proximité de l'ouverture (35) permettant de charger les récipients porte-objets (12', 13').

13. Procédé pour relier une pluralité de récipients porte-objets situés en succession dans une machine de traitement, notamment pour le lavage, la désinfection thermique et / ou la stérilisation d'objets selon l'une quelconque des revendications précédentes, ledit procédé prévoyant :le fait d'insérer un premier récipient porte-objet (12') dans une chambre de traitement (11') ; le fait d'insérer au moins un deuxième récipient porte-objet (13') dans la chambre de traitement (11') après ledit premier récipient porte-objet (12') qui a déjà été inséré dans ladite chambre de traitement (11'), et de déplacer lesdits récipients porte-objets (12', 13') vers l'intérieur de la chambre de traitement (11') ; au moins l'un desdits récipients porte-objets (12') étant pourvu d'un dispositif de connexion libérable (40, 40') activé automatiquement par des moyens d'activation (49, 49') disposés dans la chambre de traitement (11') pour connecter mutuellement lesdits récipients porte-objets (12', 13'), de manière à permettre une extraction simultanée de ceux-ci, agissant sur le premier ou le dernier récipient porte-objets (12', 13') inséré, ledit dispositif de connexion libérable (40, 40') comprenant au moins un bras rotatif (42) associé à au moins un côté (36, 37) d'un premier récipient porte-objet (12') et configuré pour passer, au moyen desdits moyens d'activation (49, 49'), d'une première position de libération inactive à une deuxième position de fixation active, et comprenant également au moins un ergot (41') dépassant d'un côté (36, 37) d'un deuxième récipient porte-objet (13') et apte à mis en relation d'engagement avec ledit bras rotatif (42) dans la position de fixation active.
